# EUROPEAN PATENT SPECIFICATION

(11) **EP 4 382 069 B1**
(45) Date of publication and mention of the grant of the patent: **25.02.2026**
(21) Application number: 21960548.2
(22) Date of filing: 11.10.2021
(51) Int. Cl.: A61B 34/30, A61B 46/10

(54) **DRAPE UNIT WITH COVER, LOCKING COVER, AND MEDICAL ROBOT**
ABDECKTUCHEINHEIT MIT ABDECKUNG, SPERRABDECKUNG UND MEDIZINISCHER ROBOTER
UNITÉ CHAMP AVEC COUVERCLE, COUVERCLE DE VERROUILLAGE ET ROBOT MÉDICAL

(43) Date of publication of application: 12.06.2024
(73) Proprietor: RIVERFIELD Inc., Tokyo 107-0052 (JP)
(72) Inventor: MORITA, Naoya, Tokyo 160-0017 (JP)
(74) Representative: Adares PartGmbB
(86) International application number: PCT/JP2021/037588
(87) International publication number: WO 2023/062687

(56) References cited:
- WO-A1-2021/166336
- WO-A1-2021/199413
- JP-A- 2008 104 854
- JP-A- 2020 163 104
- JP-A- 2020 500 606
- JP-A- 2021 129 664
- US-A1- 2016 151 115
- US-A1- 2019 167 365

## Description

### TECHNICAL FIELD

The present invention relates to a drape unit with cover including a drape unit that isolates a medical robot and a surgical instrument from each other and a locking cover, and relates also to the locking cover and a medical robot.

### BACKGROUND ART

Surgery using medical robots is attracting attention as a technique that enhances the possibility of not only reducing the burden on a surgeon but also the burden on a patient as well as the possibility of remote medical care through highly accurate and stable treatment. When using a medical robot, the surgical instrument is sterilized and therefore is a clean area, but the medical robot side is not as sterilized as the surgical instrument side and is therefore an unclear area. For this reason, in surgery using a medical robot, the medical robot side is covered with a drape in order to isolate the clean area and the unclean area from each other.

Patent Document 1 discloses a surgical system that may be configured to be a minimally invasive and/or computer assisted surgical system. Operation of the system may be controlled by transmission of the force from a first section to a second section of the system. The first section and the second section may be separated by a partition or a barrier. The first section may be a non-sterile section, and the second section may be a sterile section of the surgical system.

Patent Document 2 discloses a surgical drape that covers at least a portion of a surgical robotic arm. The drape is configured to include an interface portion that can be positioned between the arm and the instrument when the instrument is engaged with the arm, and a bulk portion that surrounds the interface portion.

Patent Document 3 discloses a medical manipulator system. The medical manipulator system comprises: a guide device having a treatment instrument channel; an insertion body passed through the treatment instrument channel; a power source section capable of being mounted to and removed from the insertion body; a guide rail having a first region corresponding to a state in which the joint section of the insertion body is located within the treatment instrument channel, the guide rail also having a second region corresponding to a state in which the joint section is exposed from the distal end of the treatment instrument channel, the guide rail guiding the power source section so that the power source section can move forward and backward in a predetermined rectilinear direction comprising the first region and the second region; and a restriction section disposed at the boundary between the first region and the second region, the boundary being located in a path for forward and backward movement of the power source section on the guide rail, the restriction section restricting the movement of the insertion body so that the insertion body does not move from the second region to the first region while the insertion body is mounted to the power source section.

Patent Document 4 discloses a drape unit disposed between a medical robot and a surgical instrument. The drape unit includes movable intervening parts that receive power from the medical robot. The surgical instrument comprises a main body to be fitted into the drape unit.

### PRIOR ART DOCUMENTS

### PATENT DOCUMENTS

Patent Document 1: JP2021-100601A
Patent Document 2: Japanese translation of PCT international application, No. 2019-523072
Patent Document 3: WO2016/125385
Patent Document 4: WO 2021/166336 A1

### OBJECT AND SUMMARY OF THE INVENTION

The portion of a medical robot to which a surgical instrument is attached is provided with a movable member that transmits driving force to the surgical instrument and an opening that guides the movement of the movable member. If such an opening is provided between the clean area and the unclean area, the isolation between the clean area and the unclean area will be insufficient.

However, in order to isolate the clean area and the unclean area from each other, it is necessary to cover the entire drive section, making it difficult to downsize the device. In addition, the drape unit, which is an intermediate member, is interposed between the medical robot and the surgical instrument, but when reattaching the drape unit to the medical robot, if it takes time to align the drive section and the movable portion of the drape unit, it may result in incorrect reattachment or it may take time for the reattachment.

An object of the present invention is to allow the clean area and unclean area to be sufficiently isolated from each other and enable the reattachment of the drape unit interposed between the medical robot and the surgical instrument to be completed accurately and in a short time.

To solve the above problems, a locking cover according to claim 1, a drape unit with cover according to claim 2, a medical robot according to claim 11, and method according to claim 13 are provided. An aspect of the present invention provides a drape unit with cover, comprising: a drape unit that is disposed between a medical robot holding a surgical instrument and the surgical instrument to isolate the surgical instrument and the medical robot
from each other, the drape unit transmitting power in a forward/backward direction from a power transmission part of the medical robot to a movable part provided in the surgical instrument; and a locking cover that can be reattached to the drape unit.

The drape unit comprises: a slider having a first engagement portion engaged with the power transmission part and a second engagement portion engaged with the movable part of the surgical instrument, the slider transmitting the power received from the power transmission part to the movable part; and a separator main body having a through-hole through which a part of the slider is inserted, the separator main body being detachably attached to the medical robot.

The locking cover comprises: a main body portion that can cover the entire through-hole; a holding portion that detachably holds a relative position of the main body portion to the separator main body; and a slider holding portion that is provided on the main body portion so as to hold a relative position between the main body portion and the slider and can be engaged with the second engagement portion.

According to such a configuration, when the locking cover is attached to the drape unit, the locking cover covers the entire through-hole provided in the separator main body, and the clean area and unclean area are isolated from each other. Moreover, when the locking cover is attached to the drape unit, the relative position of the main body portion of the locking cover with respect to the separator main body is maintained, and the relative position between the main body portion and the slider is maintained by the slider holding portion. Thus, when attaching the surgical instrument to the drape unit, positioning from the movable part of the medical robot to the movable part of the surgical instrument via the slider of the drape unit is performed accurately.

In the above drape unit with cover, the main body portion may have optical transparency, and a relative position of the slider with respect to the separator main body may be visually recognizable in a state in which the locking cover is held on the separator main body.

In the above drape unit with cover, the holding portion may have a convex portion that protrudes to the separator main body side. The drape unit may have a locking reception portion that receives the convex portion and restricts movement of the convex portion in the forward/backward direction. When the locking cover is held by the locking reception portion, the slider whose relative position is held by the slider holding portion may be engageable with the power transmission part. Through this configuration, when the locking cover is attached to the drape unit and the convex portion is received in the locking reception portion, the movement of the locking cover in the forward/backward direction is restricted, and the relative position of the slider is maintained by the slider holding portion.

In the above drape unit with cover, the drape unit may have: an initial reception portion that receives the convex portion to temporarily hold the locking cover; and a guide portion that guides the convex portion in the forward/backward direction from the initial reception portion to the locking reception portion, and the locking reception portion may be provided with a locking wall that allows the convex portion to be guided by the guide portion and move in one of forward and backward directions but restricts movement of the convex portion in the other of the forward and backward directions. Through this configuration, when the locking cover is attached to the drape unit and the convex portion is received in the initial reception portion, the relative position of the slider is maintained by the slider holding portion, and the slider can be placed at a reference position by guiding the convex portion in one direction of the forward and backward directions.

In the above drape unit with cover, when the locking cover is temporarily held by the initial reception portion, the slider holding portion may be able to engage with the second engagement portion of the slider which has moved to an end on one side of the through-hole in the forward/backward direction. When the locking cover is attached in a state in which the slider has moved to the end on one side of the through-hole in the forward/backward direction, the slider holding portion can be accurately engaged with the second engagement portion of the slider.

In the above drape unit with cover, the locking cover preferably has a symmetrical shape with a predetermined cross section as a plane of symmetry. This plane of symmetry may be a plane including a direction along the forward/backward direction of the drape unit or may also be a plane orthogonal to the forward/backward direction of the drape unit. This allows the locking cover to be attached to the drape unit regardless of the orientation in the forward/backward direction.

In the above drape unit with cover, at least one of slider holding portions is preferably equally divided by the plane of symmetry. This allows the locking cover to be attached to the drape unit regardless of the orientation in the forward/backward direction.

The present invention provides a locking cover for use with the above drape unit with cover, comprising: a main body portion that can cover the entire through-hole; a holding portion that detachably holds a relative position of the main body portion to the separator main body; and a slider holding portion that is provided on the main body portion so as to hold a relative position between the main body portion and the slider and can be engaged with the second engagement portion.

According to such a configuration, when the locking cover is attached to the drape unit, the locking cover covers the entire through-hole provided in the separator main body, and the clean area and unclean area are isolated from each other. Moreover, when the locking cover is attached to the drape unit, the relative position of the main body portion of the locking cover with respect to the separator main body is maintained, and the relative position between the main body portion and the slider is maintained by the slider holding portion. Thus, when attaching the surgical instrument to the drape unit, positioning from the movable part of the medical robot to the movable part of the surgical instrument via the slider of the drape unit is performed accurately.

Still another aspect of the present invention provides a medical robot in which the drape unit of the above drape unit with cover is disposed between the medical robot and a surgical instrument to transmit power in a forward/backward direction to the surgical instrument. The drape unit comprises: a slider having a first engagement portion engaged with the power transmission part and a second engagement portion engaged with the movable part of the surgical instrument, the slider transmitting the power received from the power transmission part to the movable part; and a separator main body having a through-hole through which a part of the slider is inserted. The separator main body is detachably attached to the medical robot. The medical robot has an attachment control part controlling an actuator part that drives the power transmission part so that the power transmission part is held at a predetermined position in the forward/backward direction when the drape unit is attached.

According to such a configuration, on the drape unit side, the relative position of the slider with respect to the separator main body is determined by attaching the locking cover, while on the medical robot side, the position of the power transmission part in the forward/backward direction is determined by control of the actuator part with the attachment control part. Therefore, when the drape unit is attached to the medical robot, the slider and the power transmission part can be engaged accurately and in a short time.

### Effect of the invention

According to the present invention, the clean area and the unclean area can be sufficiently isolated from each other, and when the drape unit interposed between the medical robot and the surgical instrument is reattached, the attachment can be completed accurately and in a short time.

### BRIEF DESCRIPTION OF DRAWINGS

FIG. 1 is a perspective view for exemplifying a medical robot and a drape unit according to an embodiment of the present invention;
FIG. 2 is a perspective view for exemplifying the attaching state of a surgical instrument;
FIG. 3 is a perspective view for exemplifying attachment of the surgical instrument to the drape unit;
FIG. 4 is a plan view for exemplifying a driving portion of the medical robot;
FIG. 5 is an exploded perspective view of the drape unit according to the present embodiment;
FIG. 6 is a cross-sectional view for exemplifying the configuration of the drape unit according to the present embodiment;
FIG. 7 is a perspective view for exemplifying a drape unit with cover;
FIG. 8A is an exploded perspective view for exemplifying the drape unit with cover;
FIG. 8B is an enlarged view of part A of FIG. 8A;
FIG. 9A is a diagram for exemplifying a locking cover;
FIG. 9B is a diagram for exemplifying the locking cover;
FIG. 10A is a schematic diagram for exemplifying a method of reattaching the drape unit;
FIG. 10B is a schematic diagram for exemplifying the method of reattaching the drape unit;
FIG. 10C is a schematic diagram for exemplifying the method of reattaching the drape unit;
FIG. 11A is a schematic diagram for exemplifying the method of reattaching the drape unit;
FIG. 11B is a schematic diagram for exemplifying the method of reattaching the drape unit;
FIG. 12A is a perspective view for exemplifying steps from reattaching the drape unit with cover to attaching the surgical instrument;
FIG. 12B is a perspective view for exemplifying steps from reattaching the drape unit with cover to attaching the surgical instrument;
FIG. 13 is a perspective view illustrating another example of the locking cover;
FIG. 14 is a plan view illustrating the other example of the locking cover;
FIG. 15 is a bottom view illustrating the other example of the locking cover;
FIG. 16 is a right side view illustrating the other example of the locking cover; and
FIG. 17 is a front view illustrating the other example of the locking cover.

### BEST MODE FOR CARRYING OUT THE INVENTION

Hereinafter, one or more embodiments of the present invention will be described with reference to the drawings. In the following description, the same members are denoted by the same reference numerals and the description of members once explained may be omitted.

### (Configuration of medical robot and drape unit)

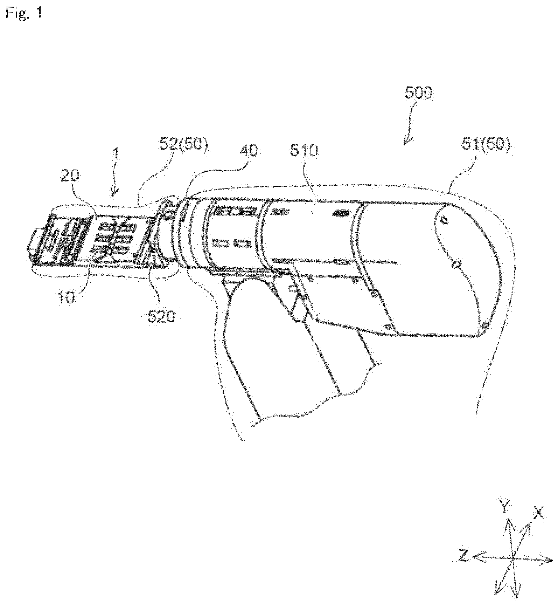

FIG. 1 is a perspective view for exemplifying a medical robot and a drape unit according to an embodiment of the present invention.

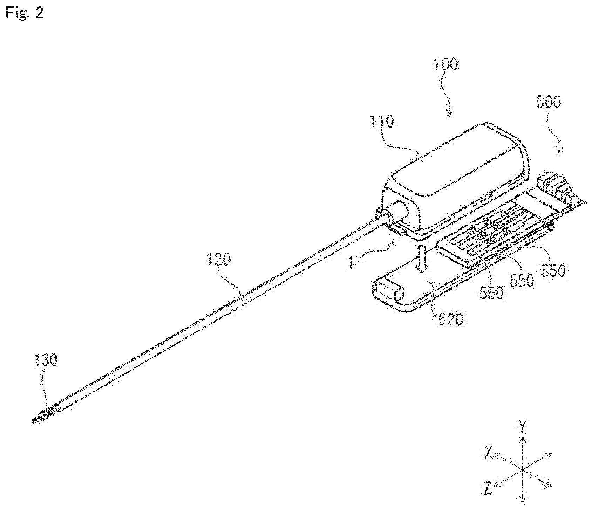

FIG. 2 is a perspective view for exemplifying the attaching state of a surgical instrument.

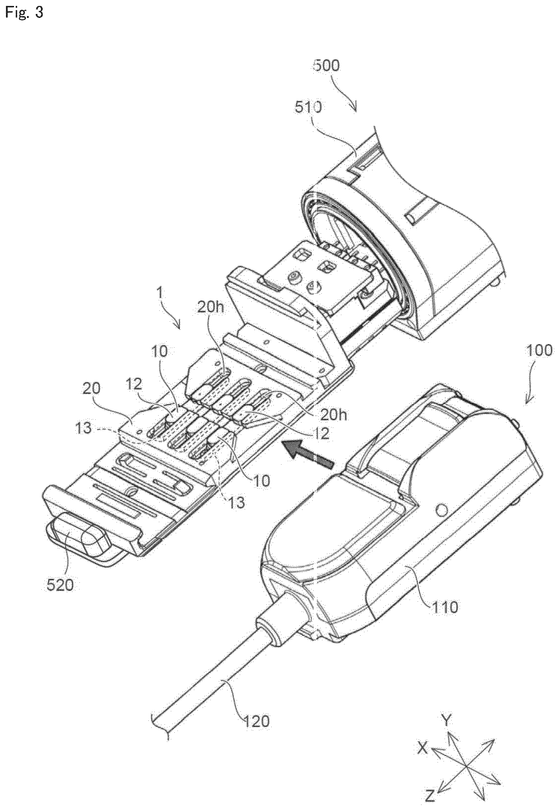

FIG. 3 is a perspective view for exemplifying attachment of the surgical instrument to the drape unit.

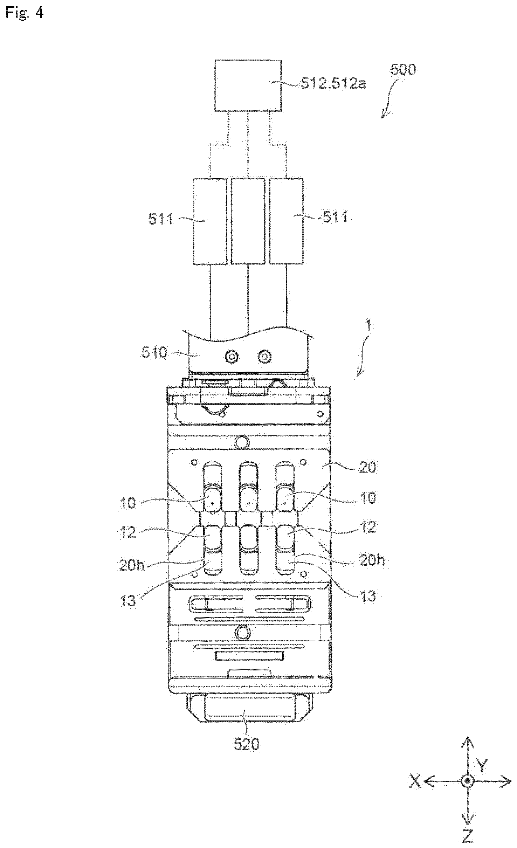

FIG. 4 is a plan view for exemplifying a driving portion of the medical robot.

As illustrated in FIGS. 1 and 2, a medical robot 500 has a multi-degree-of-freedom arm 510 that is a manipulator capable of being remotely controlled. The tip portion of the multi-degree-of-freedom arm 510 is provided with an attaching portion 520 to which a drape unit 1 according to the present embodiment is attached. A surgical instrument 100 is attached to the multi-degree-of-freedom arm 510 via the drape unit 1.

The drape unit 1 is provided with a separator main body 20. The separator main body 20 is detachably attached to the attaching portion 520 of the multi-degree-of-freedom arm 510, and the surgical instrument 100 is detachably attached to the separator main body 20 of the drape unit 1. In addition, the drape unit 1 is provided with a drape 50 that covers outside of the medical robot 500. This allows the drape unit 1 to be disposed between the medical robot 500, which holds the surgical instrument 100, and the surgical instrument 100, and the drape unit 1 serves to isolate the surgical instrument 100 and the medical robot 500 from each other. The drape unit 1 also serves to transmit power in the forward/backward direction from power transmission parts 550 of the medical robot 500 to respective movable parts 150 provided in the surgical instrument 100.

Here, in the present embodiment, the direction (forward/backward direction) of the power transmitted from the power transmission parts 550 to the drape unit 1 will be referred to as a Z direction, one of the directions orthogonal to the Z direction will be referred to as an X direction, and the direction orthogonal to the Z direction and the X direction will be referred to as a Y direction.

In the present embodiment, the Z direction is also the direction in which the tip portion of the multi-degree-of-freedom arm 510 extends. The tip portion (attaching portion 520) of the multi-degree-of-freedom arm 510 is configured to rotate about an axis in the Z direction. The arm portion of the multi-degree-of-freedom arm 510 is also axially rotatable. The surgical instrument 100 attached to the medical robot 500 can therefore approach a patient from various angles using the multi-degree-of-freedom arm 510.

As illustrated in FIG. 2, the surgical instrument 100 comprises a main body 110, a shaft 120 extending from the main body 110, and a treatment part 130 provided at the tip of the shaft 120 (an end portion opposite to the main body 110). The treatment part 130 is, for example, forceps. For easy understanding of the relationships between the surgical instrument 100 and drape unit 1 and the attaching portion 520 of the medical robot 500, FIG. 2 illustrates a situation in which the drape unit 1 in a state of being attached to the surgical instrument 100 is attached to the attaching portion 520 of the medical robot 500. At the time of actual treatment, as illustrated in FIG. 3, the drape unit 1 is first attached to the attaching portion 520 of the medical robot 500, and the surgical instrument 100 is then attached to the drape unit 1 which has been attached to the attaching portion 520.

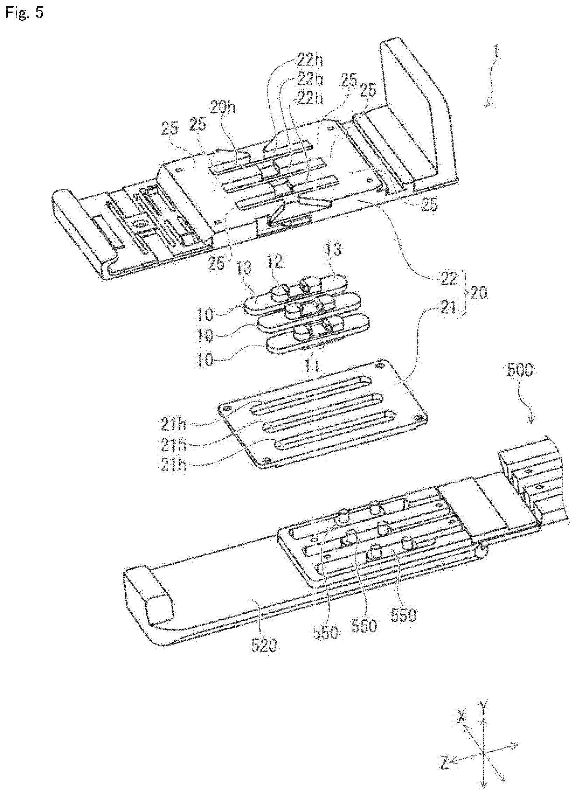

As illustrated in FIGS. 3 and 4, the medical robot 500 is provided with actuator parts 511 and a control part 512. The actuator parts 511 generate driving force for operating the surgical instrument 100. The actuator parts 511 are connected to respective power transmission parts 550 (see FIG. 5) that transmit driving force in the forward/backward direction (Z direction) to the drape unit 1.

The present embodiment will be described as being applied to an example in which the actuator parts 511 generate driving force using a gas such as air or a fluid. The actuator parts 511 may use electric motors, and the type of generating the power is not limited. The actuator parts 511 may have a configuration using a piston and a cylinder or a configuration in which the driving force is generated from other known fluids, and the specific configuration is not limited.

The control part 512 controls generation of the driving force in the actuator parts 511. The control part 512 also controls the movement in the Z direction in the power transmission parts 550 and the arrangement positions of the power transmission parts 550. The present embodiment will be described as being applied to an example in which the control part 512 controls the supply of a gas such as air to the actuator parts 511. The control part 512 also serves as an attachment control part 512a, which will be described later.

To attach the surgical instrument 100 to the multi-degree-of-freedom arm 510, the main body 110 of the surgical instrument 100 is fitted into the separator main body 20 of the drape unit 1, which has been attached to the attaching portion 520 of the multi-degree-of-freedom arm 510, so as to be slid, for example, in the X direction.

By fitting the main body 110 into the separator main body 20, the power from the power transmission parts 550 can be transmitted to the main body 110 via the drape unit 1. Link means (e.g., wires) that transmit power to the treatment part 130 are provided in the main body 110, and the movement of the power transmission parts 550 can be transmitted from the drape unit 1 to the link means of the surgical instrument 100 to operate the treatment part 130.

The surgical instrument 100 to be attached to the medical robot 500 is sterilized and placed in a clean area. On the other hand, the medical robot 500 is placed in an unclean area that is not as clean as the surgical instrument 100. The drape unit 1, which zones the clean area and the unclean area, is also sterilized.

As illustrated in FIG. 1, the drape unit 1 includes a membrane-like drape 50. The drape 50 has a first drape 51 that covers the multi-degree-of-freedom arm 510 side from a joint member 40 having a bearing structure, and a second drape 52 that covers the attaching portion 520 side from the joint member 40. The first drape 51 is covered outside of the multi-degree-of-freedom arm 510, and is connected to cover the multi-degree-of-freedom arm 510 by the joint member 40. The second drape 52 is attached so that the separator main body 20 is attached to the attaching portion 520 in a state of being covered with the first drape 51 thereby to cover the attaching portion 520 side of the joint member 40.

### (Detailed structure of drape unit)

FIG. 5 is an exploded perspective view of the drape unit according to the present embodiment.

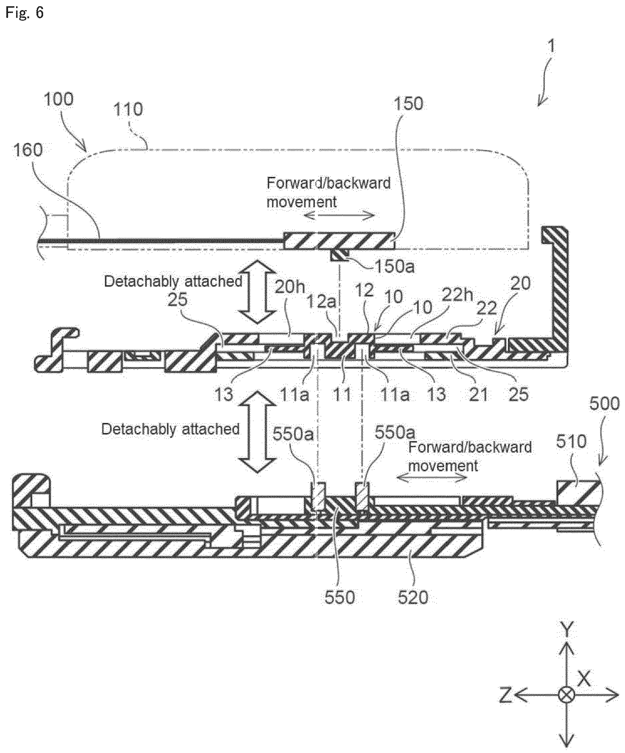

FIG. 6 is a cross-sectional view for exemplifying the configuration of the drape unit according to the present embodiment.

FIG. 6 illustrates a YZ cross section of the drape unit 1. For descriptive purposes, FIG. 6 illustrates not only the cross-sectional view of the drape unit 1 but also the cross-sectional views of the power transmission part 550 of the medical robot 500 and the movable part 150 of the surgical instrument 100, which are in a state of being isolated from the drape unit 1.

As illustrated in FIGS. 5 and 6, the drape unit 1 includes sliders 10 that receive the power from the power transmission parts 550 of the medical robot 500 and a separator main body 20 that is detachably attached to the medical robot 500. The separator main body 20 has through-holes 20h through which respective parts of the sliders 10 are inserted.

The separator main body 20 is an assembly that comprises a first fixed portion 21 and a second fixed portion 22. The sliders 10 are arranged so as to be interposed between the first fixed portion 21 and the second fixed portion 22 and are housed so as not to drop off from the separator main body 20.

The separator main body 20 has housing portions 25 that cover respective parts of the sliders 10 as viewed in the penetrating direction (Y direction) of the through-holes 20h when the sliders 10 move to end portions (movable range ends) in the forward/backward direction. That is, the housing portions 25 of the separator main body 20 are configured to house respective parts of the sliders 10 when the sliders 10 move to the movable range ends in the Z direction, and the exposure of the sliders 10 to the outside is therefore suppressed. In particular, when the surgical instrument 100 is replaced during surgery, the separator main body 20 of the drape unit 1 is in a state of being exposed from when the surgical instrument 100 attached to the medical robot 500 is detached to when another surgical instrument 100 is attached. At this time, there is a risk that the practitioner or an assistant (supporting staff) may carelessly touch the separator main body 20 or some entity (e.g., a robot arm disposed next to the separator main body 20) may collide with the separator main body 20. In the medical robot 500 according to the present embodiment, as described above, the movable range ends of the sliders 10 of the drape unit 1 in the Z direction are housed in the housing portions 25 of the separator main body 20, and therefore problems are less likely to occur, such as unexpected movement, dropping, and damage of the sliders 10 due to contact and/or collision.

Moreover, in the case of the drape unit 1 provided on the medical robot 500 as in the present embodiment, the unit provided with the power transmission parts 550 freely rotates and moves, and the through-holes 20h of the drape unit 1 face various directions accordingly. In the drape unit 1 according to the present embodiment, the sliders 10 are housed in the separator main body 20 so as not to drop off, and therefore even when the through-holes 20h of the drape unit 1 face any direction, the sliders 10 can appropriately prevent the exposure of the unclean area.

### (Detailed structure of sliders)

Each slider 10 has a first movable portion 11 that receives power from the corresponding power transmission part 550, a second movable portion 12 that transmits power to the corresponding movable part 150 of the surgical instrument 100, and a third movable portion 13 that extends from the second movable portion 12 in the forward/backward direction (Z direction).

The first movable portion 11 is provided with first engagement portions 11a into which protruding portions 550a of the power transmission part 550 are fitted. When the drape unit 1 is attached to the attaching portion 520 of the multi-degree-of-freedom arm 510, the protruding portions 550a of the power transmission part 550 are fitted into the first engagement portions 11a of the first movable portion 11 of the slider 10. This allows the power when the power transmission part 550 moves forward/backward to be transmitted to the first movable portion 11, and the slider 10 can move forward/backward.

The second movable portion 12 is provided with a second engagement portion 12a into which a protruding portion (movable part's protruding portion 150a) of the movable part 150 of the surgical instrument 100 is fitted. When the main body 110 of the surgical instrument 100 is attached to the drape unit 1, the movable part's protruding portion 150a protruding from the back surface of the main body 110 is fitted into the second engagement portion 12a of the second movable portion 12. This allows the slider 10 and the movable part 150 of the surgical instrument 100 to be engaged with each other, and the forward/backward movement of the power transmission part 550 can be transmitted from the slider 10 to the movable part 150 of the surgical instrument 100. That is, when the power transmission part 550 is moved forward/backward, the power is transmitted from the slider 10 to the movable part 150, and the forward/backward movement of the movable part 150 can be transmitted to a wire 160 to operate the treatment part 130 via the wire 160.

The third movable portion 13 is a portion that extends from the second movable portion 12 in the Z direction (forward/backward direction). When viewed in the Y direction, the third movable portion 13 is provided larger than the second movable portion 12 and therefore serves to cover the corresponding through-hole 20h.

### (Detailed structure of separator main body)

The first fixed portion 21 is provided with first openings 21h through which the first movable portions 11 of the sliders 10 are inserted, and the second fixed portion 22 is provided with second openings 22h through which the second movable portions 12 of the sliders 10 are inserted. When viewed in the Y direction, the areas in which the first openings 21h and the second openings 22h overlap are the through-holes 20h. The first openings 21h and the second openings 22h are elongated holes extending in the Z direction and guide the sliders 10 to move forward/backward in the Z direction within the range of the elongated holes.

That is, the sliders 10 can move forward/backward in the Z direction within the range of the elongated holes of the first openings 21h and the second openings 22h. In the movable regions of the sliders 10 between the first fixed portion 21 and the second fixed portion 22, portions that overlap the first fixed portion 21 as viewed in the Y direction are the housing portions 25.

### (Drape unit with cover)

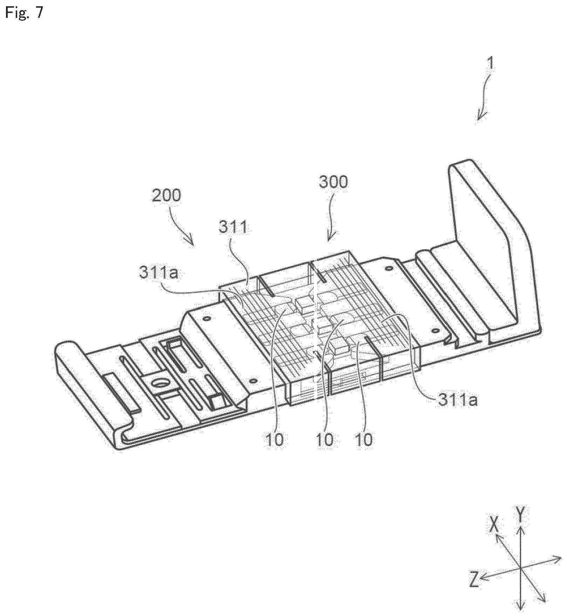

FIG. 7 is a perspective view for exemplifying a drape unit with cover.

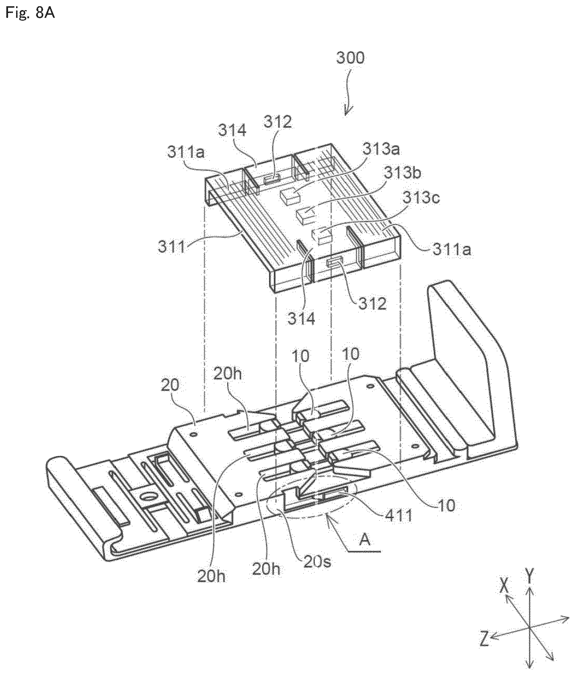

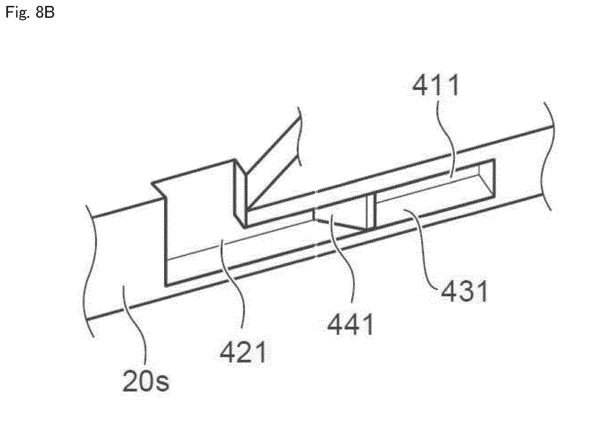

FIG. 8A is an exploded perspective view for exemplifying the drape unit with cover, and FIG. 8B is an enlarged view of part A of FIG. 8A.

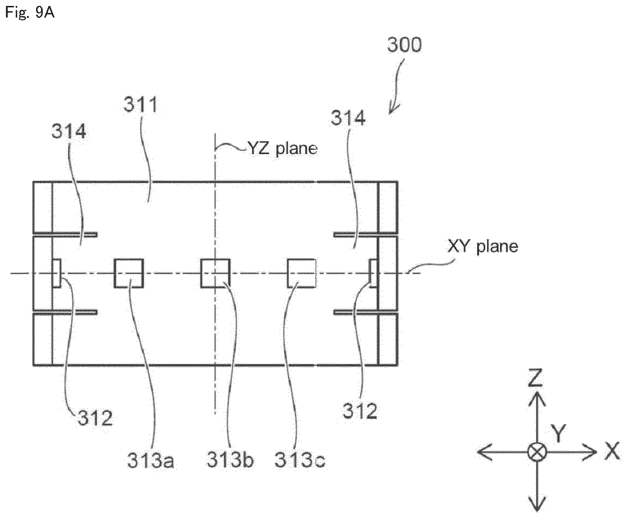

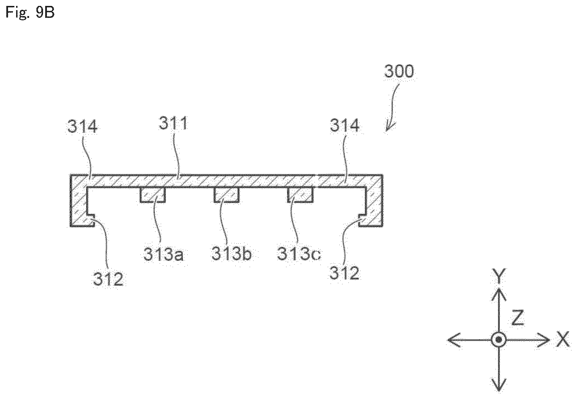

FIGS. 9A and 9B are diagrams for exemplifying a locking cover. FIG. 9A illustrates a plan view of a locking cover 300 as viewed from the back surface, and FIG. 9B illustrates a cross-sectional view of the locking cover.

A drape unit with cover 200 includes the previously described drape unit 1 and a locking cover 300 that can be reattached to the drape unit 1. The drape unit with cover 200 is applied when the drape unit 1 is attached to or reattached to the attaching portion 520 of the medical robot 500.

In the drape unit with cover 200 in which the locking cover 300 is attached to the drape unit 1, the locking cover 300 covers the entire through-holes 20h provided in the separator main body 20, and the isolation between the clean area and the unclean area can therefore be ensured.

When the drape unit 1 is attached to the medical robot 500, the drape 50 may be jammed by mistake, or the drape unit 1 may not be attached at the correct position, resulting in incorrect attachment. In such a case, the drape unit 1 is once detached from the attaching portion 520 and attached again (reattached) after dealing with the jamming or reconsidering the attaching position.

When the drape unit 1 is reattached, the relative positions of the sliders 10 with respect to the separator main body 20 are maintained by attaching the locking cover 300 to the drape unit 1, and the drape unit 1 can be attached accurately in a short time.

### (Locking cover)

The locking cover 300 includes a main body portion 311, holding portions 314, and slider holding portions 313a, 313b, and 313c. When the slider holding portions 313a, 313b, and 313c are collectively referred to without distinction, they will be referred to as slider holding portions 313.

The main body portion 311 is a plate-shaped member that can cover the entire through-holes 20h when the locking cover 300 is attached to the separator main body 20 of the drape unit 1. The surface of the main body portion 311 may be provided with protrusions 311a. By providing the protrusions 311a, when the locking cover 300 is slid on the separator main body 20, the locking cover 300 is easily caught on a finger, and the sliding operation is facilitated. A specific example of the protrusion height of the protrusions 311a is 0.1 mm or more and 0.5 mm or less, and a more specific example is 0.25 mm or more and 0.35 mm or less.

The holding portions 314 are vertical pieces provided at both end portions of the main body portion 311 in the width direction, and are portions that detachably hold the relative position of the main body portion 311 with respect to the separator main body 20. When the locking cover 300 is attached to the separator main body 20, the holding portions 314 engage with the separator main body 20 so as to embrace both side surfaces 20s of the separator main body 20.

Specifically, the holding portions 314 are provided in a state of having spring properties relative to the main body portion 311, and convex portions 312 protruding inward are provided at positions of the holding portions 314 facing the side surfaces 20s of the separator main body 20. On the other hand, both the side surfaces 20s of the separator main body 20 of the drape unit 1 are provided with locking reception portions 411 that receive the convex portions 312 to restrict movement of the convex portions 312 in the forward/backward direction (Z direction).

The slider holding portions 313 engage with the second engagement portions 12a of the sliders 10 when the locking cover 300 is attached to the separator main body 20. In the present embodiment, the slider holding portions 313a, 313b, and 313c engage with respective second engagement portions 12a of three sliders 10. By engaging the slider holding portions 313 with the second engagement portions 12a, the relative positions between the main body portion 311 and the sliders 10 are maintained.

Therefore, when the locking cover 300 is placed over the separator main body 20 and the convex portions 312 of the holding portions 314 are fitted into the locking reception portions 411 to hold the locking cover 300, the slider holding portions 313 maintain the relative positions of the sliders 10. This allows the sliders 10 to be accurately engaged with the protruding portions 550a of the power transmission parts 550 in the first engagement portions 11a.

Each side surface 20s of the separator main body 20 is provided with an initial reception portion 421 and a guide portion 431. The initial reception portion 421 receives the convex portion 312 provided on the holding portion 314 to temporarily hold the locking cover 300. The guide portion 431 guides the convex portion 312 in the forward/backward direction from the initial reception portion 421 to the locking reception portion 411. In addition, the locking reception portion 411 is provided with a locking wall 441.

The initial reception portion 421, guide portion 431, and locking reception portion 411 are formed, for example, by grooves provided in the side surface 20s of the separator main body 20. The grooves extend in the forward/backward direction (Z direction), and the initial reception portion 421 is arranged on one side in the forward/backward direction while the locking reception portion 411 is arranged on the other side. The locking wall 441 is provided on the initial reception portion 421 side of the locking reception portion 411.

When the convex portion 312 is engaged with the initial reception portion 421 to temporarily hold the locking cover 300, each slider holding portion 313 can engage with the second engagement portion 12a of the corresponding slider 10 which has moved to an end on one side of the through-hole 20h in the forward/backward direction.

The locking wall 441 has an inclined surface that becomes higher in the direction from the initial reception portion 421 toward the locking reception portion 411 (away from the side surface 20s in the X direction). Such a locking wall 441 allows the convex portion 312 to be guided to the guide portion 431 and move in one of the forward and backward directions (direction from the initial reception portion 421 toward the locking reception portion 411), but can restrict the movement of the convex portion 312 in the other direction (the direction from the locking reception portion 411 toward the initial reception portion 421).

The main body portion 311 of the locking cover 300 preferably has optical transparency. This makes it easier to visually recognize the relative positions of the sliders 10 with respect to the separator main body 20 in a state in which the locking cover 300 is held by the separator main body 20.

As illustrated in FIG. 9A, the locking cover 300 preferably has a symmetrical shape with a predetermined cross section as a plane of symmetry. This plane of symmetry may be a plane (YZ plane) including a direction along the forward/backward direction (Z direction) or a plane (XY plane) orthogonal to the forward/backward direction (Z direction). This allows the locking cover 300 to be attached to the drape unit 1 regardless of the orientation in the forward/backward direction.

### (Method of reattaching drape unit)

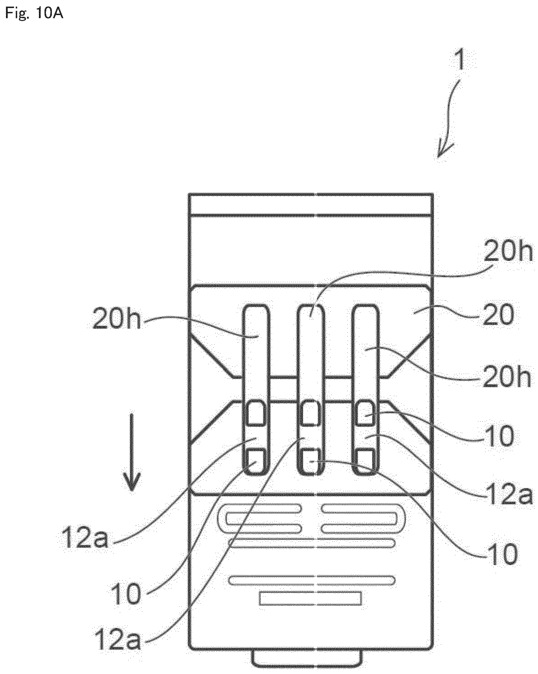

FIGS. 10A to 11B are schematic diagrams for exemplifying a method of reattaching the drape unit.

First, as illustrated in FIG. 10A, in a state in which the drape unit 1 is detached from the attaching portion 520, the sliders 10 are moved to an end on one side of the separator main body 20. In the state in which the drape unit 1 is detached from the attaching portion 520, the sliders 10 and the power transmission parts 550 are not engaged, so the sliders 10 are free. Therefore, by turning the distal end side of the separator main body 20 downward, for example, the sliders 10 move downward by their own weights and move to the lowest positions along the through-holes 20h.

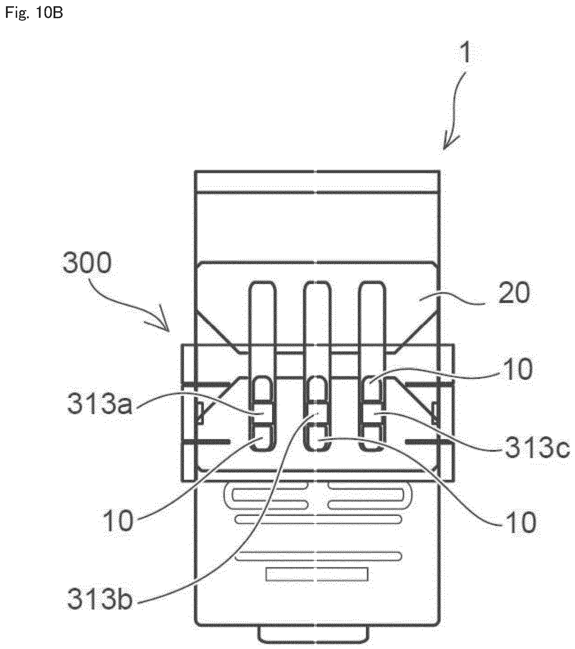

Then, as illustrated in FIG. 10B, the locking cover 300 is attached to the separator main body 20. In this attaching, the convex portions 312 provided on the holding portions 314 of the locking cover 300 are engaged with the initial reception portions 421 to temporarily hold the locking cover 300. In the state in which the locking cover 300 is temporarily held, the slider holding portions 313 of the locking cover 300 are engaged with the second engagement portions 12a of the sliders 10, which have moved to the end on one side of the separator main body 20, and the sliders 10 can be held in a state of being positioned at the end on one side of the separator main body 20.

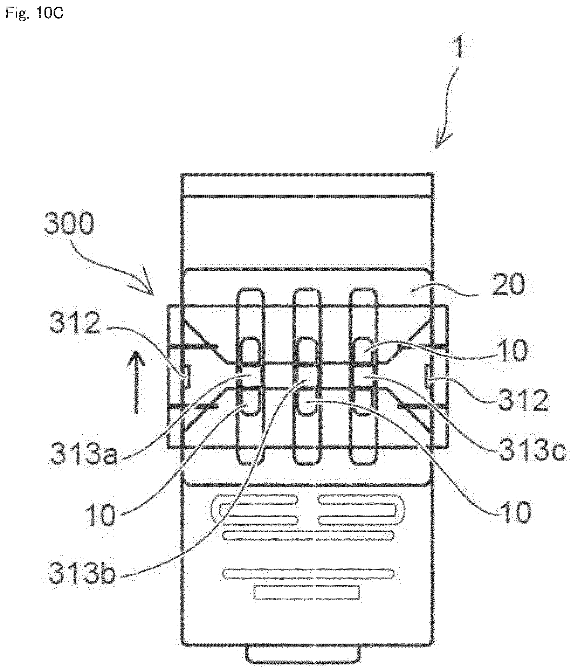

Next, as illustrated in FIG. 10C, the position of the locking cover 300 relative to the separator main body 20 is slid to the other side in the forward/backward direction. In this operation, when the protrusions 311a (see FIGS. 7 and 8A) are provided on the surface of the main body portion 311 of the locking cover 300, the locking cover 300 is easily caught on a finger, and the sliding operation is facilitated. By this sliding operation, the convex portions 312 of the holding portions 314 are guided from the initial reception portions 421 to the guide portions 431 over the locking walls 441 and reach the locking reception portions 411. This allows the relative position of the locking cover 300 with respect to the separator main body 20 to be fixed. In this operation, as the locking cover 300 slides, the sliders 10 engaging with the slider holding portions 313 also move. For example, when the convex portions 312 are received in the locking reception portions 411 and the locking cover 300 is fixed at a predetermined position, the sliders 10 are placed at neutral positions in the forward/backward direction through the through-holes 20h.

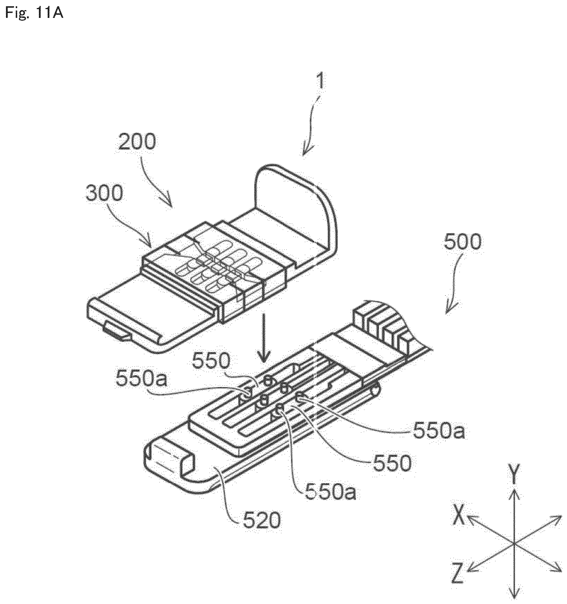

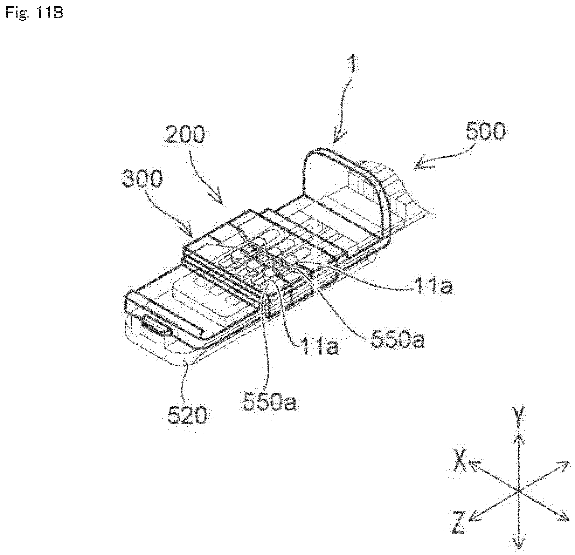

Then, as illustrated in FIG. 11A, the drape unit with cover 200 to which the locking cover 300 is fixed at the predetermined position is reattached to the attaching portion 520 of the medical robot 500. In this operation, the actuator parts 511 which drive the power transmission parts 550 are controlled by the control part 512 so that the power transmission parts 550 are held at predetermined positions in the forward/backward direction. For example, the control part 512 illustrated in FIG. 4 is made to serve as the attachment control part 512a, and the actuator parts 511 are controlled to move the power transmission parts 550 to predetermined positions (e.g., neutral positions) in the forward/backward direction. When the drape unit with cover 200 is attached to the attaching portion 520 in this state, the protruding portions 550a of the power transmission parts 550 can be accurately engaged with the first engagement portions 11a of the sliders 10 of the drape unit 1 (see FIG. 11B).

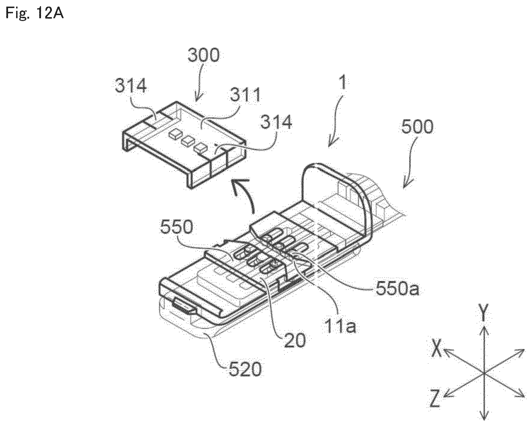

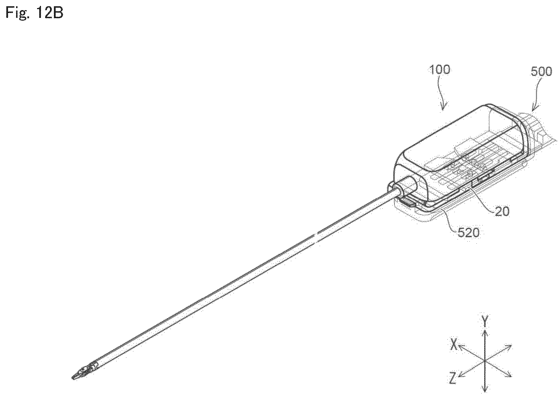

FIGS. 12A and 12B are perspective views for exemplifying steps from reattaching the drape unit with cover to attaching the surgical instrument.

As illustrated in FIG. 12A, after the drape unit with cover 200 is reattached to the attaching portion 520 of the medical robot 500, the locking cover 300 is detached. Even when the locking cover 300 is detached, the position of the slider 10 does not shift because the first engagement portions 11a of the sliders 10 and the protruding portions 550a of the power transmission parts 550 are engaged. Here, the locking cover 300 is designed to be easily detached along the XY plane. To this end, the holding portions 314 are provided in a cantilever shape with cuts in the X direction relative to the main body portion 311. Therefore, by hooking one holding portion 314 with the thumb, for example, and pulling it up along the XY plane, the one holding portion 314 can be detached so as to rotate about the other holding portion 314 as a fulcrum. This allows the locking cover 300 to be easily detached with one hand.

Then, as illustrated in FIG. 12B, the surgical instrument 100 is attached to the separator main body 20 of the drape unit 1. In this operation, since all the sliders 10 of the separator main body 20 are arranged at the neutral positions, the second engagement portions 12a of the sliders 10 are aligned in the X direction. This allows the surgical instrument 100 to be attached to an accurate position by sliding the main body 110 of the surgical instrument 100 in the X direction and fitting it in (see FIG. 3).

Thus, when the drape unit 1 is reattached, the locking cover 300 is attached to the drape unit 1, which has been once detached from the attaching portion 520, and is temporarily held, and the locking cover 300 is slid thereby to allow all the sliders 10 to be aligned at predetermined positions (e.g., neutral positions) with respect to the separator main body 20. This allows the positions of the sliders 10, which are free because the drape unit 1 is detached, to be maintained and aligned at the positions required for reattaching, and when the drape unit with cover 200 is reattached to the attaching portion 520, the first engagement portions 11a of the sliders 10 and the protruding portions 550a of the power transmission parts 550 can be engaged accurately and in a short time. It is therefore possible to reattach the drape unit 1 while maintaining hygiene without touching the drape unit 1 unnecessarily.

### (Another example of locking cover)

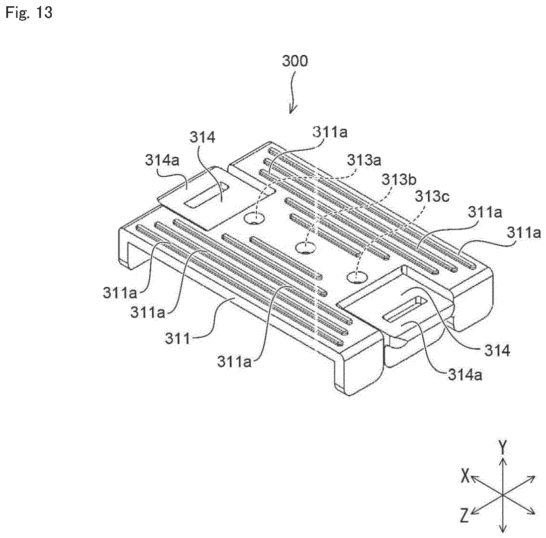

FIG. 13 is a perspective view illustrating another example of the locking cover.

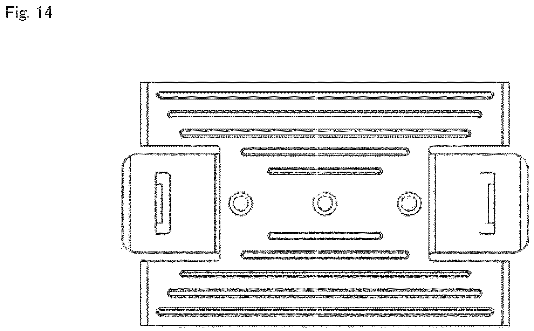

FIG. 14 is a plan view illustrating the other example of the locking cover.

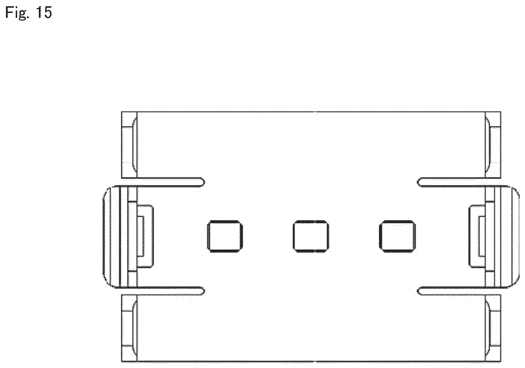

FIG. 15 is a bottom view illustrating the other example of the locking cover.

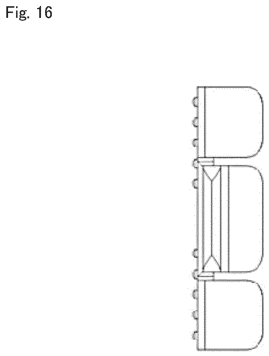

FIG. 16 is a right side view illustrating the other example of the locking cover.

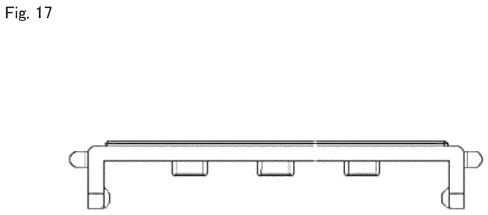

FIG. 17 is a front view illustrating the other example of the locking cover.

In the other example of the locking cover 300, the left side view is symmetrical with the right side view, and the rear view is symmetrical with the front view.

In the other example of the locking cover 300, the holding portions 314 provided at both end portions of the main body portion 311 in the width direction are provided with extending portions 314a. The extending portions 314a are provided so as to extend from the corner portions of the holding portions 314 in the X direction. When the holding portions 314 are provided at both end portions of the main body portion 311 in the width direction, the extending portions 314a extending outward in the X direction from the corner portions of respective holding portions 314 are provided.

The extending length of the extending portions 314a may be a length that allows fingers to be easily caught on the extending portions 314a. A specific example of the extending length of the extending portions 314a is 1 mm or more and 5 mm or less, and a more specific example is 2 mm or more and 3 mm or less. Each extending portion 314a may be provided over the entire area of the holding portion 314 in the Z direction, or may also be provided in a part of the holding portion 314 in the Z direction.

Provision of the extending portions 314a results in an effect as follows when the locking cover 300 attached to the attaching portion 520 of the medical robot 500 is detached. That is, when the extending portion 314a of one of the holding portions 314 is hooked, for example, with a thumb to pull it up along the XY plane, the locking cover 300 can be easily detached by rotating it using the other holding portion 314 as a fulcrum.

As described above, according to the present embodiment, it is possible to provide the drape unit with cover 200, the locking cover 300, and the medical robot 500 with which the clean area and the unclean area can be sufficiently isolated from each other, and when the drape unit interposed between the medical robot and the surgical instrument is reattached, the attachment can be completed accurately and in a short time.

Although the present embodiments have been described above, the present invention is not limited to these examples. For example, in the above embodiments, an example in which three sliders 10 are arranged in parallel has been described, but the number of the sliders 10 is not limited. Moreover, an example of the forceps has been described as the treatment part 130 of the surgical instrument 100, but a treatment part 130 other than the forceps may be employed. Furthermore, the scope of the present invention encompasses those to which a person skilled in the art appropriately makes addition or removal of constitutional elements or design changes with respect to the previously-described embodiments and those in which features of the embodiments are appropriately combined, provided that they fall within the scope of protection defined by the wording of the claims.

### DESCRIPTION OF REFERENCE NUMERALS

- 1: Drape unit
- 10: Slider
- 11: First movable portion
- 11a: First engagement portions
- 12: Second movable portion
- 12a: Second engagement portion
- 13: Third movable portion
- 20: Separator main body
- 20h: Through-hole
- 20s: Side surface
- 21: First fixed portion
- 21h: First opening
- 22: Second fixed portion
- 22h: Second opening
- 25: Housing portion
- 40: Joint member
- 50: Drape
- 51: First drape
- 52: Second drape
- 100: Surgical instrument
- 110: Main body
- 120: Shaft
- 130: Treatment part
- 150: Movable part
- 150a: Movable part's protruding portion
- 160: Wire
- 200: Drape unit with cover
- 300: Locking cover
- 311: Main body portion
- 311a: Protrusion
- 312: Convex portion
- 313, 313a, 313b, 313c: Slider holding portion
- 314: Holding portion
- 314a: Extending portions
- 411: Locking reception portion
- 421: Initial reception portion
- 431: Guide portion
- 441: Locking wall
- 500: Medical robot
- 510: Multi-degree-of-freedom arm
- 511: Actuator part
- 512: Control part
- 512a: Attachment control part
- 520: Attaching portion
- 550: Power transmission part
- 550a: Protruding portion

## Claims

1. A locking cover (300) for use with a drape unit (1) with cover (200), wherein the drape unit (1) is configured to transmit power in a forward/backward direction from a power transmission part (550) of a medical robot (500) to a movable part (150) provided in a surgical instrument (100) and wherein the drape unit (1) comprises a slider (10) having a first engagement portion (11a) configured to engage with the power transmission part (550) and a second engagement portion (12a) configured to engage with the movable part (150) of the surgical instrument (100), the slider (10) configured to transmit the power received from the power transmission part (550) to the movable part (150), and wherein the drape unit (1) comprises a separator main body (20) having a through-hole (20h) through which a part of the slider (10) is inserted,
wherein the locking cover (300) comprises:
a main body portion (311) configured to cover the entire through-hole (20h);
a holding portion (314) configured to detachably hold a relative position of the main body portion (311) to the separator main body (20) when a convex portion (312) of the holding portion (314) is fitted into locking reception portions (411) of the separator main body (20); and
a slider holding portion (313a, 313b, 313c) that is provided on the main body portion (311) so as to hold a relative position between the main body portion (311) and the slider (10) and can be engaged with the second engagement portion (12a).

2. A drape unit with cover (200), comprising:
a drape unit (1) configured to be disposed between a medical robot (500) holding a surgical instrument (100) and the surgical instrument (100) to isolate the surgical instrument (100) and the medical robot (500) from each other, the drape unit (1) configured to transmit power in a forward/backward direction from a power transmission part (550) of the medical robot (500) to a movable part (150) provided in the surgical instrument (100); and
the drape unit (1) comprising:
a slider (10) having a first engagement portion (11a) configured to engage with the power transmission part (550) and a second engagement portion (12a) configured to engage with the movable part (150) of the surgical instrument (100), the slider (10) configured to transmit the power received from the power transmission part (550) to the movable part (150); and
a separator main body (20) having a through-hole (20h) through which a part of the slider (10) is inserted, the separator main body (20) configured to be detachably attached to the medical robot (500); the drape unit with cover (200) further comprising a locking cover (300) according to claim 1 which is configured to be reattached to the drape unit (1).

3. The drape unit with cover according to claim 2, wherein the main body portion (311) has optical transparency, and a relative position of the slider (10) with respect to the separator main body (20) can be visually recognized in a state in which the locking cover (300) is held on the separator main body (20).

4. The drape unit with cover according to claim 2 or 3, wherein
the holding portion (314) has a convex portion (312) that protrudes to the separator main body (20) side,
the drape unit (1) has a locking reception portion (411) configured to receive the convex portion (312) and restrict movement of the convex portion (312) in the forward/backward direction, and
when the locking cover (300) is held by the locking reception portion (411), the slider (10) whose relative position is held by the slider holding portion (313) can be engaged with the power transmission part.

5. The drape unit with cover according to claim 4, wherein
the drape unit (1) has:
an initial reception portion (421) configured to receive the convex portion (312) to temporarily hold the locking cover (300); and
a guide portion (431) configured to guide the convex portion (312) in the forward/backward direction from the initial reception portion (421) to the locking reception portion (411), and
the locking reception portion (411) is provided with a locking wall (441) configured to allow the convex portion (312) to be guided by the guide portion (431) and move in one of forward and backward directions but restricts movement of the convex portion (312) in the other of the forward and backward directions.

6. The drape unit with cover according to claim 5, wherein when the locking cover (300) is temporarily held by the initial reception portion (421), the slider holding portion (313) is configured to engage with the second engagement portion (12a) of the slider (10) which has moved to an end on one side of the through-hole (20h) in the forward/backward direction.

7. The drape unit with cover according to any one of claims 2 to 6, wherein the locking cover (300) has a symmetrical shape with a predetermined cross section as a plane of symmetry.

8. The drape unit with cover according to claim 7, wherein in a state in which the locking cover (300) is attached to the drape unit (1), the plane of symmetry is a plane including a direction along the forward/backward direction of the drape unit (1).

9. The drape unit with cover according to claim 7 or 8, wherein in a state in which the locking cover (300) is attached to the drape unit (1), the plane of symmetry is a plane orthogonal to the forward/backward direction of the drape unit (1).

10. The drape unit with cover according to any one of claims 7 to 9, wherein at least one of slider holding portions (313a, 313b, 313c) is equally divided by the plane of symmetry.

11. A medical robot (500) comprising the drape unit with cover (200) according to any one of claims 2 to 10
the drape unit (1) comprising the slider (10) having the first engagement portion (11a) engaged with the power transmission part (550) ;
the medical robot (500) having an attaching portion (520) to which the drape unit (1) is attached, wherein the locking cover (300) is attached to the separator main body (20),
the medical robot (500) having an attachment control part (512a) configured to control an actuator part (511) configured to drive the power transmission part (550) so that the power transmission part (550) is held at a predetermined position in the forward/backward direction when the drape unit (1) is attached.

12. The medical robot according to claim 11, wherein the power transmission part (550) has a protruding portion (550a) accurately engaged with the first engagement portion (11a) of the slider (10) of the drape unit (1).

13. A method for attaching a surgical instrument (100) to a medical robot (500), in which the method comprises the following steps:
a drape unit with cover (200) according to any one of claims 2 to 10 is attached to the medical robot (500) to establish a state where the locking cover (300) is attached but a surgical instrument (100) is not yet mounted;
the locking cover (300) is removed from the medical robot (500);
and after the removal of the locking cover (300) from the medical robot (500), the surgical instrument (100) is mounted to the medical robot (500).

14. The method according to claim 13, wherein the drape unit with cover (200) is attached to an attaching portion (520) of the medical robot (500), and after the removal of the locking cover (300) from the medical robot (500), the surgical instrument (100) is mounted to the medical robot (500) by attachment to the separator main body (20) of the drape unit (1).

15. The method according to any one of claims 13 or 14, wherein the removing of the locking cover (300) is made by hooking one holding portion (314) and pulling it up along a plane so as to rotate about the other holding portion (314) as a fulcrum, whereas the first engagement portion (11a) of the slider (10) and the protruding portion (550a) of the power transmission part (550) are engaged after the removal of the locking cover (300).

## Patentansprüche

1. Verriegelungsabdeckung (300) zur Verwendung mit einer Drape-Einheit (1) mit Abdeckung (200), wobei die Drape-Einheit (1) konfiguriert ist, Strom in einer Vorwärts-/Rückwärtsrichtung von einem Stromübertragungsteil (550) eines medizinischen Roboters (500) zu einem beweglichen Teil (150) zu übertragen, das in einem chirurgischen Instrument (100) bereitgestellt ist, und wobei die Drape-Einheit (1) einen Schieber (10) mit einem ersten Eingriffsabschnitt (11a), der konfiguriert ist, mit dem Stromübertragungsteil in Eingriff zu stehen (550), und einem zweiten Eingriffsabschnitt (12a) enthält, der konfiguriert ist, mit dem beweglichen Teil (150) des chirurgischen Instruments (100) in Eingriff zu stehen, wobei der Schieber (10) konfiguriert ist, den vom Stromübertragungsteil empfangenen Strom (550) an den beweglichen Teil (150) zu übertragen, und wobei die Drape-Einheit (1) einen Separator-Hauptkörper (20) mit einem Durchgangsloch (20h) enthält, durch das ein Teil des Schiebers (10) eingeführt wird,
wobei die Verriegelungsabdeckung (300) enthält:
einen Hauptkörperabschnitt (311), der konfiguriert ist, das gesamte Durchgangsloch (20 H) abzudecken;
einen Halteabschnitt (314), der konfiguriert ist, abnehmbar eine relative Position des Hauptkörperabschnitts (311) zum Separator-Hauptkörper (20) zu halten, wenn ein konvexer Abschnitt (312) des Halteabschnitts (314) in Verriegelungsaufnahmeabschnitte (411) des Separator-Hauptkörpers (20) eingepasst wird; und
einen Schieber-Halteabschnitt (313a, 313b, 313c), der am Hauptkörperabschnitt (311) bereitgestellt ist, um eine relative Position zwischen dem Hauptkörperabschnitt (311) und dem Schieber (10) zu halten, und mit dem zweiten Eingriffsabschnitt (12a) in Eingriff gebracht werden kann.

2. Drape-Einheit mit Abdeckung (200), enthaltend:
eine Drape-Einheit (1), die konfiguriert ist, zwischen einem medizinischen Roboter (500), der ein chirurgisches Instrument (100) hält, und dem chirurgischen Instrument (100) angeordnet zu werden, um das chirurgische Instrument (100) und den medizinischen Roboter (500) voneinander zu isolieren, wobei die Drape-Einheit (1) konfiguriert ist, Strom in eine Vorwärts-/Rückwärtsrichtung von einem Stromübertragungsteil (550) des medizinischen Roboters (500) zu einem beweglichen Teil (150), das im chirurgischen Instrument (100) bereitgestellt ist, zu übertragen; und
wobei die Drape-Einheit (1) enthält:
einen Schieber (10) mit einem ersten Eingriffsabschnitt (11a), der konfiguriert ist, mit dem Stromübertragungsteil (550) in Eingriff zu stehen, und einem zweiten Eingriffsabschnitt (12a), der konfiguriert ist, mit dem beweglichen Teil (150) des chirurgischen Instruments (100) in Eingriff zu stehen, wobei der Schieber (10) konfiguriert ist, den vom Stromübertragungsteil empfangenen Strom (550) an den beweglichen Teil (150) zu übertragen; und
einen Separator-Hauptkörper (20) mit einem Durchgangsloch (20H), durch das ein Teil des Schiebers (10) eingeführt ist; wobei der Separator-Hauptkörper (20) konfiguriert ist, abnehmbar am medizinischen Roboter (500) befestigt zu werden, wobei die Drape-Einheit mit Abdeckung (200) weiterhin eine Verriegelungsabdeckung (300) gemäß Anspruch 1 enthält, die konfiguriert ist, an der Drape-Einheit (1) wieder befestigt zu werden.

3. Drape-Einheit mit Abdeckung gemäß Anspruch 2, wobei der Hauptkörperabschnitt (311) optische Transparenz aufweist, und eine relative Position des Schiebers (10) in Bezug auf den Separator-Hauptkörper (20) visuell erkannt werden kann in einem Zustand, in dem die Verriegelungsabdeckung (300) am Separator-Hauptkörper gehalten wird (20).

4. Drape-Einheit mit Abdeckung gemäß Anspruch 2 oder 3, wobei
der Halteabschnitt (314) einen konvexen Abschnitt (312) aufweist, der zur Seite des Separator-Hauptkörpers (20) vorspringt,
die Drape-Einheit (1) einen Verriegelungsaufnahmeabschnitt (411) aufweist, der konfiguriert ist, den konvexen Abschnitt (312) aufzunehmen und eine Bewegung des konvexen Abschnitts (312) in Vorwärts-/Rückwärtsrichtung einzuschränken, und
wenn die Verriegelungsabdeckung (300) vom Verriegelungsaufnahmeabschnitt (411) gehalten wird, der Schieber (10), dessen relative Position vom Verriegelungshalteabschnitt (313) gehalten wird, mit dem Stromübertragungsteil in Eingriff gebracht werden kann.

5. Drape-Einheit mit Abdeckung gemäß Anspruch 4, wobei
wobei die Drape-Einheit (1) aufweist:
einen Anfangsaufnahmeabschnitt (421), der konfiguriert ist, den konvexen Abschnitt (312) aufzunehmen, um die Verriegelungsabdeckung temporär zu halten (300); und
einen Führungsabschnitt (431), der konfiguriert ist, den konvexen Abschnitt (312) in der Vorwärts-/Rückwärtsrichtung vom Anfangsaufnahmeabschnitt (421) zum Verriegelungsabschnitt (411) zu führen, und
der Verriegelungsaufnahmeabschnitt (411) mit einer Verriegelungswand (441) bereitgestellt ist, die konfiguriert ist, dem konvexen Abschnitt (312) zu erlauben, vom Führungsabschnitt (431) geführt zu werden und sich in eine der Vorwärts- und Rückwärtsrichtungen zu bewegen, aber eine Bewegung des konvexen Abschnitts (312) in der anderen der Vorwärts- und Rückwärtsrichtungen einzuschränken.

6. Drape-Einheit mit Abdeckung gemäß Anspruch 5, wobei die Verriegelungsabdeckung (300) vom Anfangsaufnahmeabschnitt (421) temporär gehalten wird, der Schieberhalteabschnitt (313) konfiguriert ist, mit dem zweiten Eingriffsabschnitt (12a) des Schiebers (10) in Eingriff zu stehen, der sich in der Vorwärts-/Rückwärtsrichtung zu einem Ende an einer Seite des Durchgangslochs (20h) bewegt hat.

7. Drape-Einheit mit Abdeckung gemäß einem der Ansprüche 2 bis 6, wobei die Verriegelungsabdeckung (300) eine symmetrische Form mit einem vorgegebenen Querschnitt als eine Symmetrieebene aufweist.

8. Drape-Eeinheit mit Abdeckung gemäß Anspruch 7, wobei in einem Zustand, in dem die Verriegelungsabdeckung (300) an der Drape-Einheit (1) befestigt ist, die Symmetrieebene eine Ebene ist, die eine Richtung entlang der Vorwärts-/Rückwärtsrichtung der Drape-Einheit (1) aufweist.

9. Drape-Einheit mit Abdeckung gemäß Anspruch 7 oder 8, wobei in einem Zustand, in dem die Verriegelungsabdeckung (300) an der Drape-Einheit (1) befestigt ist, die Symmetrieebene eine Ebene ist, die orthogonal zur Vorwärts-/Rückwärtsrichtung der Drapie-Einheit (1) ist.

10. Drape-Einheit mit Abdeckung gemäß einem der Ansprüche 7 bis 9, wobei mindestens einer der Schieberhalteabschnitte (313a, 313b, 313c) gleichmäßig durch die Symmetrieebene geteilt wird.

11. Medizinischer Roboter (500), enthaltend die Drape-Einheit mit Abdeckung (200) gemäß einem der Ansprüche 2 bis 10
wobei die Drape-Einheit (1) den Schieber (10) enthält, der den ersten Eingriffsabschnitt (11a) aufweist, der mit dem Stromübertragungsteil (550) in Eingriff steht;
wobei der medizinische Roboter (500) einen Befestigungsabschnitt (520) aufweist, an dem die Drape-Einheit (1) befestigt ist, wobei die Verriegelungsabdeckung (300) am Separator-Hauptkörper (20) befestigt ist,
wobei der medizinische Roboter (500) einen Befestigungssteuerungsteil (512A) aufweist, der konfiguriert ist, einen Aktuatorteil (511) zu steuern, der konfiguriert ist, den Stromübertragungsteil (550) anzutreiben, sodass der Stromübertragungsteil (550) in einer vorgegebenen Position in der Vorwärts-/Rückwärtsrichtung gehalten wird, wenn die Drape-Einheit (1) befestigt ist.

12. Medizinischer Roboter gemäß Anspruch 11, wobei der Stromübertragungsteil (550) einen vorspringenden Abschnitt (550a) aufweist, der mit dem ersten Eingriffsabschnitt (11a) des Schiebers (10) der Drape-Einheit (1) präzise in Eingriff steht.

13. Verfahren zum Befestigen eines chirurgischen Instruments (100) an einem medizinischen Roboter (500), wobei das Verfahren die folgenden Schritte enthält:
eine Drape-Einheit mit Abdeckung (200) gemäß einem der Ansprüche 2 bis 10 wird am medizinischen Roboter (500) befestigt, um einen Zustand zu etablieren, in dem die Verriegelungsabdeckung (300) befestigt ist, aber ein chirurgisches Instrument (100) noch nicht montiert ist;
die Verriegelungsabdeckung (300) wird vom medizinischen Roboter (500) entfernt;
und nach dem Entfernen der Verriegelungsabdeckung (300) vom medizinischen Roboter (500) wird das chirurgische Instrument (100) am medizinischen Roboter (500) montiert.

14. Verfahren gemäß Anspruch 13, wobei die Drape-Einheit mit Abdeckung (200) an einem Befestigungsabschnitt (520) des medizinischen Roboters (500) befestigt wird, und nach dem Entfernen der Verriegelungsabdeckung (300) vom medizinischen Roboter (500) das chirurgische Instrument (100) am medizinischen Roboter (500) durch Befestigung am Separator-Hauptkörper (20) der Drape-Einheit (1) befestigt wird.

15. Verfahren gemäß einem der Ansprüche 13 oder 14, wobei das Entfernen der Verriegelungsabdeckung (300) durchgeführt wird durch Einhaken eines Halteabschnitts (314) und Ziehen von ihm entlang einer Ebene nach oben, sodass er sich um den anderen Halteabschnitt (314) als ein Drehpunkt dreht, während der erste Eingriffsabschnitt (11a) des Schiebers (10) und der vorspringende Abschnitt (550a) des Stromübertragungsteils (550) nach dem Entfernen des Verriegelungsdeckung (300) in Eingriff gebracht werden.

## Revendications

1. Un couvercle verrouillable (300) pour une utilisation avec une unité de champ (1) avec un couvercle (200), dans lequel l'unité de champ (1) est configurée pour transmettre une énergie dans une direction avant/arrière depuis une part de transmission d'énergie (550) d'un robot médical (500) vers une part mobile (150) fournie dans un instrument chirurgical (100) et dans lequel l'unité de champ (1) comprend un curseur (10) avec une première partie d'engagement (11a) configurée pour s'engager avec la part de transmission d'énergie (550) et une seconde partie d'engagement (12a) configurée pour s'engager avec la part mobile (150) de l'instrument chirurgical (100), le curseur (10) configuré pour transmettre l'énergie reçue de la part de transmission d'énergie (550) vers la part mobile (150), et dans lequel l'unité de champ (1) comprend un corps principal de séparateur (20) avec un trou traversant (20h) par lequel une part du curseur (10) est insérée,
dans lequel le couvercle verrouillable (300) comprend :
une partie principale (311) configurée pour couvrir tout le trou traversant (20h) ;
une partie de maintien (314) configurée pour maintenir de manière détachable une position relative de la partie principale du corps principal (311) par rapport au corps principal de séparateur (20) lorsqu'une partie convexe (312) de la partie de maintien (314) est intégrée dans les parties de réception verrouillables (411) du corps principal de séparateur (20) ; et
une partie de maintien du curseur (313A, 313B, 313C) qui est fournie sur la partie du corps principal (311) afin de maintenir une position relative entre la partie du corps principal (311) et le curseur (10) et peut être engagée avec la seconde partie d'engagement (12A).

2. Une unité de champ avec un couvercle (200), comprenant :
une unité de champ (1) configurée pour être disposée entre un robot médical (500) tenant un instrument chirurgical (100) et l'instrument chirurgical (100) pour isoler l'instrument chirurgical (100) et le robot médical (500) l'un de l'autre, l'unité de champ (1) configurée pour transmettre l'énergie dans une direction avant/arrière depuis une part de transmission d'énergie (550) du robot médical (500) vers une part mobile (150) fournie dans l'instrument chirurgical (100); et
dans lequel l'unité de champ (1) comprend :
un curseur (10) ayant une première partie d'engagement (11a) configurée pour s'engager avec la part de transmission d'énergie (550) et une seconde partie d'engagement (12a) configurée pour s'engager avec la part mobile (150) de l'instrument chirurgical (100), le curseur (10) configuré pour transmettre l'énergie reçue de la part de transmission d'énergie (550) vers la part mobile (150) ; et
un corps principal de séparateur (20) comportant un trou traversant (20h) par lequel une part du curseur (10) est insérée, le corps principal de séparateur (20) configuré pour être fixé de manière détachable au robot médical (500), l'unité de champ avec le couvercle comprenant un couvercle verrouillable (300) selon la revendication 1, qui est configuré pour être rattaché à l'unité de champ (1).

3. L'unité de champ avec le couvercle selon la revendication 2, dans laquelle la partie du corps principal (311) a une transparence optique, et une position relative du curseur (10) par rapport au corps principal de séparateur (20) peut être reconnue visuellement dans un état dans lequel le couvercle verrouillable (300) est maintenu sur le corps principal de séparateur (20).

4. L'unité de champ avec le couvercle selon la revendication 2 ou 3, dans laquelle
la partie de maintien (314) a une portion convexe (312) qui dépasse du côté du corps principal de séparateur (20),
l'unité de champ (1) a une partie de réception verrouillable (411) configurée pour recevoir la partie convexe (312) et restreindre un mouvement de la partie convexe (312) dans la direction avant/arrière, et
lorsque le couvercle de verrouillage (300) est maintenu par la partie de réception verrouillable (411), le couvercle (10) dont la position relative est maintenue par la partie de maintien du curseur (313) peut être engagé avec la part de transmission d'énergie.

5. L'unité de champ avec le couvercle selon la revendication 4, dans laquelle
l'unité de champ (1) a :
une partie de réception initiale (421) configurée pour recevoir la partie convexe (312) afin de maintenir temporairement le couvercle verrouillable (300) ; et
une portion guide (431) configurée pour guider la partie convexe (312) dans la direction avant/arrière de la partie de réception initiale (421) à la partie de réception verrouillable (411), et
la partie de réception verrouillable (411) est équipée d'un mur verrouillable (441) configuré pour permettre à la partie convexe (312) d'être guidée par la partie guide (431) et de se déplacer dans l'une des directions avant et arrière, mais restreindre le mouvement de la partie convexe (312) dans l'autre direction avant et arrière.

6. L'unité de champ avec le couvercle selon la revendication 5, dans laquelle lorsque le couvercle verrouillable (300) est temporairement maintenu par la partie de réception initiale (421), la partie de maintien du curseur (313) est configurée pour s'engager avec la seconde partie d'engagement (12a) du curseur (10) qui s'est déplacée à une extrémité d'un côté du trou traversant (20h) dans la direction avant/arrière.

7. L'unité de champ avec le couvercle selon l'une des revendications 2 à 6, dans laquelle le couvercle verrouillable (300) a une forme symétrique avec une section transversale prédéterminée comme plan de symétrie.

8. L'unité de champ avec le couvercle selon la revendication 7, dans laquelle dans un état dans lequel le couvercle verrouillable (300) est fixé à l'unité de champ (1), le plan de symétrie est un plan incluant une direction le long de la direction avant/arrière de l'unité de champ (1).

9. L'unité de champ avec le couvercle selon la revendication 7 ou 8, dans laquelle un état dans lequel le couvercle verrouillable (300) est fixé à l'unité de champ (1), le plan de symétrie est un plan orthogonal à la direction avant/arrière de l'unité de champ (1).

10. L'unité de champ avec le couvercle selon l'une des revendications 7 à 9, dans laquelle au moins une des parties de maintien du curseur (313a, 313b, 313c) est divisée également par le plan de symétrie.

11. Un robot médical (500) comprenant l'unité de champ avec le couvercle (200) selon chacune des revendications 2 à 10 ;
l'unité de champ (1) comprenant le curseur (10) ayant la première partie d'engagement (11A) engagée avec la part de transmission d'énergie (550) ;
le robot médical (500) ayant une partie d'attache (520) à laquelle est fixée l'unité de champ (1), dans lequel le couvercle verrouillable (300) est fixé au corps principal de séparateur (20),
le robot médical (500) ayant une part de contrôle d'attache (512A) configurée pour contrôler une part actionneure (511) configurée pour entraîner la part de transmission d'énergie (550) de sorte que la part de transmission d'énergie (550) soit maintenue à une position prédéterminée dans la direction avant/arrière lorsque l'unité de champ (1) est fixée.

12. Le robot médical selon la revendication 11, dans lequel la part de transmission d'énergie (550) a une partie saillante (550a) engagée avec précision avec la première partie d'engagement (11a) du curseur (10) de l'unité de champ (1).

13. Une méthode pour fixer un instrument chirurgical (100) à un robot médical (500), dans laquelle la méthode comprend les étapes suivantes :
une unité de champ avec un couvercle (200) selon l'une des revendications 2 à 10 est fixée au robot médical (500) pour établir un état dans lequel le couvercle verrouillable (300) est fixé mais un instrument chirurgical (100) n'est pas encore monté ;
le couvercle verrouillable (300) est retiré du robot médical (500) ;
et après le retrait du couvercle verrouillable (300) du robot médical (500), l'instrument chirurgical (100) est monté sur le robot médical (500).

14. La méthode selon la revendication 13, dans laquelle l'unité de champ avec le couvercle (200) est fixée à une partie d'attache (520) du robot médical (500), et après le retrait du couvercle verrouillable (300) du robot médical (500), l'instrument chirurgical (100) est monté sur le robot médical (500) par fixation au corps principal de séparateur (20) de l'unité de champ (1).

15. "La méthode, selon l'une des revendications 13 ou 14, dans laquelle le retrait du couvercle de verrouillage (300) est fait en accrochant une partie de maintien (314) et en la tirant vers le haut le long d'un plan afin de tourner autour de l'autre partie de maintien (314) comme point d'appui, tandis que la première partie d'engagement (11a) du curseur (10) et la partie saillante (550a) de la part de transmission d'énergie (550) sont engagées après le retrait du couvercle verrouillable (300).
